# EUROPÄISCHE PATENTSCHRIFT

(11) **EP 0 709 379 B1**
(45) Veröffentlichungstag und Bekanntmachung des Hinweises auf die Patenterteilung: **14.01.1998**
(21) Anmeldenummer: 95116034.0
(22) Anmeldetag: 11.10.1995
(51) Int. Cl.: C07D 263/04, C07D 263/10

(54) **Verfahren zur Herstellung von in einer Seitenkette fluorierten Alkyloxazol-Derivaten**
Process for the preparation of alkyloxazole derivatives fluorinated on a side chain
Procédé de préparation de dérivés d'alkyloxazole fluorés sur une chaîne latérale

(30) Priorität: 24.10.1994 DE 4437932
(43) Veröffentlichungstag der Anmeldung: 01.05.1996
(73) Patentinhaber: BAYER AG, 51368 Leverkusen (DE)
(72) Erfinder: Lui, Norbert, Dr., D-51061 Köln (DE); Marhold, Albrecht, Dr., D-51373 Leverkusen (DE)

(56) Entgegenhaltungen:
- WO-A-90/02739
- WO-A-94/14764
- BULL. CHEM. SOC. JAPAN, Bd. 52, Nr. 11, November 1979 Seiten 3377-3380, A. TAKAOKA ET AL.

## Beschreibung

Die vorliegende Erfindung betrifft ein besonders vorteilhaftes Verfahren zur Herstellung von fluorierten Alkyl oxazol-Derivaten, die als Zwischenprodukte für die Herstellung von Antibiotika gegen grampositive, gramnegative und gegen Thiamphenicol resistente Mikroorganismen von Bedeutung sind (siehe z.B. US-A 4 235 892).

Aus der US-A 4 876 352 ist bekannt, daß man bestimmte Hydroxyalkyloxazolin-Derivate mit α,α-Difluoralkylaminen unter Druck zu den entsprechenden Fluor-Derivaten umsetzen kann. Geeignete Drucke sind dabei z.B. solche von 60 bis 100 psi (ca. 4,2 bis 7 bar); als Lösungsmittel gelangt insbesondere Methylenchlorid zum Einsatz (siehe dort die Ansprüche 2 und 5 und die Beispiele). Nachteilig ist die Arbeitsweise unter Druck, die besonderen technischen Aufwand erfordert und der bevorzugte Einsatz von Methylenchlorid, das aus ökologischen Gründen besondere Arbeitsschutzmaßnahmen erfordert.

Es wurde nun ein Verfahren zur Herstellung von in einer Seitenkette fluorierten Alkyloxazol-Derivaten der Formel (I) gefunden 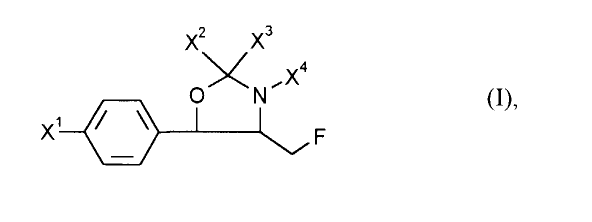 in der
- X¹: für Wasserstoff, Nitro, C₁-C₆-Alkylthio, C₁-C₆-Alkylsulfo, mit Fluor substituiertes C₁-C₆-Alkylthio, mit Fluor substituiertes C₁-C₆-Alkylsulfonyl oder mit Fluor substituiertes C₁-C₆-Alkoxy,
- X²: für C₁-C₆-Alkyl, C₁-C₆-Halogenalkyl, C₅-C₇-Cycloalkyl, C₂-C₆-Alkenyl, C₂-C₆-Alkinyl, C₁-C₆-Alkoxy, C₇-C₁₄-Aralkyl, C₈-C₁₅-Aralkenyl, C₆-C₁₂-Aryl oder einen heterocyclischen Rest mit 4 bis 11 C-Atomen und 1 bis 3 Sauerstoff-, Schwefel- und/oder Stickstoffatomen,
- X³: für Wasserstoff oder einen der bei X² angegebenen Reste oder
X² und X³ gemeinsam für ein Sauerstoffatom und
- X⁴: für Wasserstoff oder
X³ und X⁴ gemeinsam für eine kovalente Bindung stehen,
bei dem man das entsprechende Hydroxyalkyloxazol-Derivat der Formel (II) 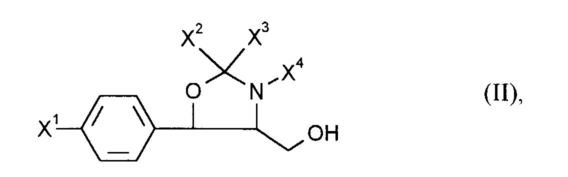 in der die Reste X¹ bis X⁴ die bei Formel (I) angegebene Bedeutung haben,
mit einem α,α-Difluoralkylamin der Formel (III) umsetzt 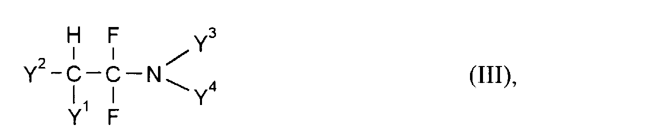 in der
- Y¹: für Fluor oder Chlor,
- Y²: für Fluor, Chlor oder Trifluormethyl und
- Y³ und Y⁴: unabhängig voneinander für C₁-C₆-Alkyl stehen oder
Y³ und Y⁴ gemeinsam für eine gesättigte oder ungesättigte Kohlenwasserstoffkette mit 4 bis 5 C-Atomen stehen,
das dadurch gekennzeichnet ist, daß man bei Temperaturen im Bereich 70 bis 150°C bei Drucken im Bereich 0,1 bis l bar und in Gegenwart eines inerten, bei über 100°C (bei Normaldruck) siedenden Lösungsmittels ausgewählt aus der Gruppe Tetramethylensulfon, Chlorbenzol, Dichlorbenzole und Trichlorbenzole arbeitet.

Die Verbindungen der Formel (II) mit X¹ = Wasserstoff, Nitro, C₁-C₆-Alkylthio oder C₁-C₆-Alkylsulfo können beispielsweise gemäß der EP-A1 130 633 oder J. Chem. Soc. 1949, 589-594 oder analog dazu und die Verbindungen der Formel (III) beispielsweise gemäß US-A 3 153 644 oder Bull. Chem. Soc. Japan 1979, 3377 bis 3380 oder analog dazu erhalten werden. Die sonstigen Verbindungen der Formel (II) können analog US-A 2 816 915 und EP-A1 130 633 oder J. Chem. Soc. 1949, 589-594 hergestellt werden.

In den Formeln (I) und (II) stehen
X¹ vorzugsweise für C₁-C₃-Alkylsulfonyl, insbesondere Methylsulfonyl,
X² vorzugsweise für Phenyl oder Naphthyl, insbesondere Phenyl und
X³ und X⁴ vorzugsweise gemeinsam für eine kovalente Bindung.

Bevorzugte Verbindungen der Formel (III) sind:
N-(2-Chlor-1,1,2-trifluorethyl)-diethylamin,
N-(1,1,2,3,3,3-Hexafluorpropyl)-diethylamin und
N-(1,1,2,3,3,3-Hexafluorpropyl)-dibutylamin.

Bezogen auf 1 Mol einer Verbindung der Formel (II) kann man beispielsweise 0,8 bis 5 Äquivalente einer Verbindung der Formel (III) einsetzen. Vorzugsweise liegt diese Menge bei 1,3 bis 3 Äquivalenten.

Das erfindungsgemäße Verfahren wird bei Normaldruck durchgeführt.

Bevorzugt sind Temperaturen im Bereich 90 bis 120°C.

Gegebenenfalls kann die erfindungsgemäße Umsetzung unter einer inerten Gasatmosphäre, z.B. Stickstoff, durchgeführt werden.

Das nach der erfindungsgemäßen Umsetzung vorliegende Reaktionsgemisch kann man beispielsweise aufarbeiten, indem man es mit Wasser verdünnt, in eine organische und eine wäßrige Phase trennt, die wäßrige Phase mit einem mit Wasser nicht mischbaren organischen Lösungsmittel extrahiert, beispielsweise einem mit Wasser nicht mischbaren Ether, die organische Phase mit Wasser wäscht, die organische Phase und das organische Extrakt aus der wäßrigen Phase vereinigt und, gegebenenfalls nach Trocknung, das Lösungsmittel entfernt, z.B. durch Destillation im Vakuum.

Das erfindungsgemäße Verfahren hat den Vorteil, daß es die gemäß der US-A 4 876 352 unbedingt erforderliche Arbeitsweise unter Druck vermeidet und trotzdem die Verbindungen der Formel (I) in guten Ausbeuten liefert.

### Beispiele

### Beispiel 1

20 g D-threo-(-)-2-Phenyl-4-(4-(methylsulfonyl)phenyl)-2-oxazolinyl-5-methanol und 17,5 g N-(1,1,2,3,3,3-hexafluorpropyl)diethylamin in 150 ml Chlorbenzol wurden auf 100°C erhitzt und 3,5 Stunden bei dieser Temperatur gerührt. Die Reaktionsmischung wurde mit Wasser verdünnt, die organische Phase abgetrennt, die wäßrige Phase mit Methyl-tert.-butylether extrahiert, die organische Phase mit Wasser gewaschen und die organische Phase und das Extrakt aus der wäßrigen Phase vereinigt. Nach dessen Trocknung über Natriumsulfat wurde das Lösungsmittel im Vakuum entfernt. In einer Ausbeute von 85 % d.Th. wurde D-threo-(-)-2-Phenyl-4-(fluormethyl)-5-(4-methylsulfonyl)-phenyl-2-oxazolin erhalten (bestimmt durch HPLC).

### Beispiel 2

10 g D-threo-(-)-2-Phenyl-4-(4-(methylsulfonyl)phenyl)-2-oxazolinyl-5-methanol und 15 g N-(1,1,2,3,3,3-hexafluorpropyl)diethylamin in 150 ml Tetramethylensulfon wurde wie in Beispiel 1 beschrieben aufgearbeitet. Es wurden 10 g der gleichen Verbindung wie in Beispiel 1 erhalten, was einer Ausbeute von 82 % d.Th. entspricht.

### Beispiel 3

10 g D-threo-(-)-2-Phenyl-4-(4-(methylsulfonyl)phenyl)-2-oxazolinyl-5-methanol und 15 g N-(1,1,2,3,3,3-hexafluorpropyl)diethylamin in 120 ml eines Dichlorbenzol-Isomerengemischs wurden auf 110°C erhitzt und 4 Stunden bei dieser Temperatur gerührt. Die Aufarbeitung des Reaktionsgemisches erfolgte wie in Beispiel 1 beschrieben. Es wurde die gleiche Verbindung wie in Beispiel 1 in einer Ausbeute von 79 % d.Th. erhalten.

## Patentansprüche

1. Verfahren zur Herstellung von in einer Seitenkette fluorierten Alkyloxazol-Derivaten der Formel 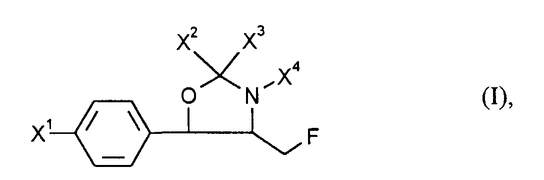 in der
X¹ für Wasserstoff, Nitro, C₁-C₆-Alkylthio, C₁-C₆-Alkylsulfo, mit Fluor substituiertes C₁-C₆-Alkylthio, mit Fluor substituiertes C₁-C₆-Alkylsulfonyl oder mit Fluor substituiertes C₁-C₆-Alkoxy,
X² für C₁-C₆-Alkyl, C₁-C₆-Halogenalkyl, C₅-C₇-Cydoalkyl, C₂-C₆-Alkenyl, C₂-C₆-Alkinyl, C₁-C₆-Alkoxy, C₇-C₁₄-Aralkyl, C₈-C₁₅-Aralkenyl, C₆-C₁₂-Aryl oder einen heterocyclischen Rest mit 4 bis 11 C-Atomen und 1 bis 3 Sauerstoff-, Schwefel- und/oder Stickstoffatomen,
X³ für Wasserstoff oder einen der bei X² angegebenen Reste oder
X² und X³ gemeinsam für ein Sauerstoffatom und
X⁴ für Wasserstoff oder
X³ und X⁴ gemeinsam für eine kovalente Bindung stehen,
bei dem man das entsprechende Hydroxyalkyloxazol-Derivat der Formel (II) 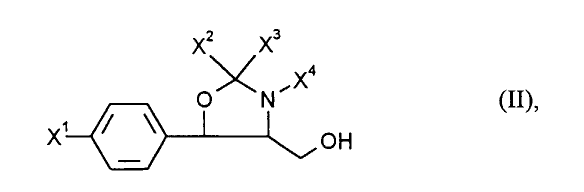 in der die Reste X¹ bis X⁴ die bei Formel (I) angegebene Bedeutung haben,
mit einem α,α-Difluoralkylamin der Formel (III) umsetzt 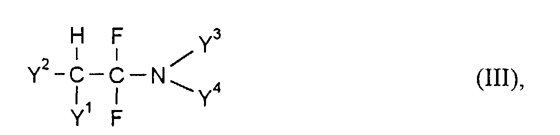 in der
Y¹ für Fluor oder Chlor,
Y² für Fluor, Chlor oder Trifluormethyl und
Y³ und Y⁴ unabhängig voneinander für C₁-C₆-Alkyl stehen oder
Y³ und Y⁴ gemeinsam für eine gesättigte oder ungesättigte Kohlenwasserstoffkette mit 4 bis 5 C-Atomen stehen,
dadurch gekennzeichnet, daß man bei Temperaturen im Bereich 70 bis 150°C und bei Normaldruck und in Gegenwart eines inerten, bei über 100°C (bei Normaldruck) siedenden Lösungsmittels ausgewählt aus der Gruppe Tetramethylensulfon, Chlorbenzol, Dichlorbenzole oder Trichlorbenzole arbeitet.

2. Verfahren nach Anspruch 1, dadurch gekennzeichnet, daß in den Formeln (I) und (II)
X¹ für C₁-C₃-Alkylsulfonyl,
X² für Phenyl oder Naphthyl und
X³ und X⁴ gemeinsam für eine kovalente Bindung stehen.

3. Verfahren nach Ansprüchen 1 und 2, dadurch gekennzeichnet, daß man bezogen auf 1 Mol einer Verbindung der Formel (II) 0,8 bis 5 Äquivalente einer Verbindung der Formel (III) einsetzt.

## Claims

1. Process for the preparation of side chain-fluorinated alkyloxazole derivatives of the formula 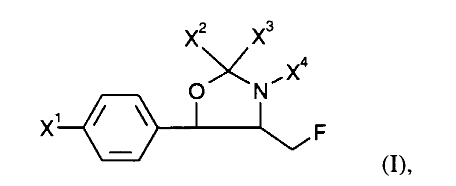 in which
X¹ represents hydrogen, nitro, C₁-C₆-alkylthio, C₁-C₆-alkylsulpho, C₁-C₆-alkylthio substituted by fluorine, C₁-C₆-alkylsulphonyl substituted by fluorine or C₁-C₆-alkoxy substituted by fluorine,
X² represents C₁-C₆-alkyl, C₁-C₆-halogenoalkyl, C₅-C₇-cycloalkyl, C₂-C₆-alkenyl, C₂-C₆-alkinyl, C₁-C₆-alkoxy, C₇-C₁₄-aralkyl, C₈-C₁₅-aralkenyl, C₆-C₁₂-aryl or a heterocyclic radical having 4 to 11 C atoms and 1 to 3 oxygen, sulphur and/or nitrogen atoms,
X³ represents hydrogen or one of the radicals indicated in the case of X² or
X² and X³ together represent an oxygen atom and
X⁴ represents hydrogen or
X³ and X⁴ together represent a covalent bond,
in which the appropriate hydroxyalkyloxazole derivative of the formula (II) 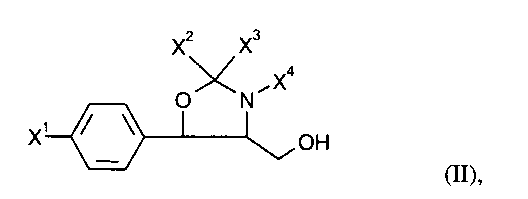 in which the radicals X¹ to X⁴ have the meaning indicated in formula (I),
is reacted with an α,α-difluoroalkylamine of the formula (III) 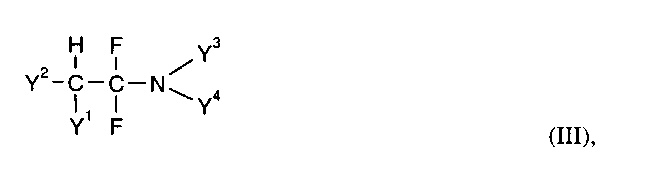 in which
Y¹ represents fluorine or chlorine,
Y² represents fluorine, chlorine or trifluoromethyl and
Y³ and Y⁴ independently of one another represent C₁-C₆-alkyl or
Y³ and Y⁴ together represent a saturated or unsaturated hydrocarbon chain having 4 to 5 C atoms,
characterized in that the reaction is carried out at temperatures in the range from 70 to 150°C and at normal pressure and in the presence of an inert solvent boiling at above 100°C (at normal pressure) and selected from the group consisting of tetramethylene sulphone, chlorobenzene, dichlorobenzenes or trichlorobenzenes.

2. Process according to Claim 1, characterized in that, in the formulae (I) and (II),
X¹ represents C₁-C₃-alkylsulphonyl,
X² represents phenyl or naphthyl and
X³ and X⁴ together represent a covalent bond.

3. Process according to Claims 1 and 2, characterized in that, relative to 1 mol of a compound of the formula (II), 0.8 to 5 equivalents of a compound of the formula (III) are employed.

## Revendications

1. Procédé pour la préparation de dérivés d'alkyloxazoles fluorés dans une chaîne latérale, répondant à la formule 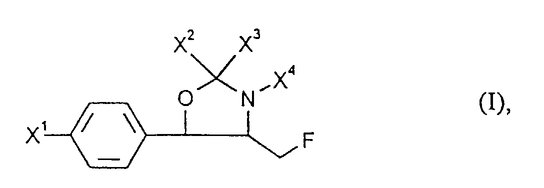 dans laquelle
X¹ représente un atome d'hydrogène, un groupe nitro, un groupe alkyl (en C₁-C₆)thio, un groupe alkyl(en C₁-C₆)sulfo, un groupe alkyl(en C₁-C₆)thio portant un ou plusieurs substituants fluoro, un groupe alkyl(en C₁-C₆)sulfonyle portant un ou plusieurs substituants fluoro, un groupe alcoxy en C₁-C₆ portant un ou plusieurs substituants fluoro,
X² représente un groupe alkyle en C₁-C₆, un groupe halogénalkyle en C₁-C₆, un groupe cycloalkyle en C₅-C₇, un groupe alcényle en C₂-C₆, un groupe alcynyle en C₂-C₆, un groupe alcoxy en C₁-C₆, un groupe aralkyle en C₇-C₁₄, un groupe aralcényle en C₈-C₁₅, un groupe aryle en C₆-C₁₂ ou encore un radical hétérocyclique contenant de 4 à 11 atomes de carbone et de i à 3 atomes d'oxygène, de soufre et/ou d'azote,
X³ représente un atome d'hydrogène ou un des radicaux indiqués dans X², ou bien
X² et X³ représentent ensemble un atome d'oxygène et
X⁴ représente un atome d'hydrogène, ou bien
X³ et X⁴ représentent ensemble une liaison covalente,
dans lequel on fait réagir le dérivé d'hydroxyalkyloxazole correspondant répondant à la formule (II) 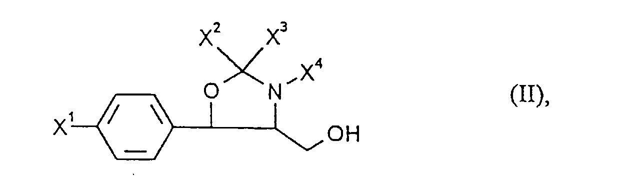 dans laquelle les radicaux X¹ à X⁴ ont la signification indiquée dans la formule (I),
avec une α,α-difluoroalkylamine de formule (III) 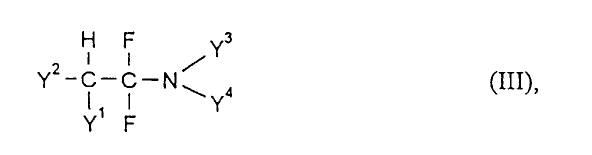 dans laquelle
Y¹ représente un atome de fluor ou un atome de chlore,
Y² représente un atome de fluor, un atome de chlore ou un groupe trifluorométhyle, et
Y³ et Y⁴ représentent, indépendamment l'un de l'autre, un groupe alkyle en C₁-C₆, ou bien
Y³ et Y⁴ représentent ensemble une chaîne d'hydrocarbures saturée ou insaturée contenant de 4 à 5 atomes de carbone,
caractérisé en ce qu'on travaille à des températures dans le domaine de 70 à 150°C et sous pression normale et en présence d'un solvant inerte dont le point d'ébullition est supérieur à 100°C (sous pression normale), choisi parmi le groupe comprenant la tétraméthylènesulfone, le chlorobenzène, les dichlorobenzènes ou les trichlorobenzènes.

2. Procédé selon la revendication 1, caractérisé en ce que, dans les formules (I) et (II),
X¹ représente un groupe alkyl(en C₁-C₃)sulfonyle,
X² représente un groupe phényle ou un groupe naphtyle, et
X³ et X⁴ représentent ensemble une liaison covalente.

3. Procédé selon les revendications 1 et 2, caractérisé en ce qu'on met en oeuvre, pour 1 mole d'un composé de formule (II), de 0,8 à 5 équivalents d'un composé de formule (III).
